# EUROPEAN PATENT APPLICATION

(11) **EP 4 624 562 A1**
(43) Date of publication of application: **01.10.2025**
(21) Application number: 25164194.0
(22) Date of filing: 17.03.2025
(51) Int. Cl.: C12M 1/42, C12M 1/34

(54) **CELL PUNCTURE DEVICE AND MICROSCOPE SYSTEM**

(30) Priority: 29.03.2024 JP 2024057962
(71) Applicant: Yokogawa Electric Corporation, Tokyo 180-8750 (JP)
(72) Inventor: KUWATA, Masahiro, Musashino-shi, Tokyo, 180-8750 (JP); FUKUDA, Takumi, Musashino-shi, Tokyo, 180-8750 (JP)
(74) Representative: Stöckeler, Ferdinand

(57) **Abstract**

A cell puncture device 10 according to the present disclosure includes a needle unit 17 having a needle 17a configured to puncture a cell S and a needle fixer 17b configured to fix the needle 17a, a needle support 18a that is connected to the needle fixer 17b and that is configured to arrange the needle unit 17 at a distal end, and a first regulator 18b that is arranged with respect to the needle support 18a and that is configured to locate the needle unit 17 in a first position in which the needle 17a punctures the cell S. The needle support 18a includes a mover 18a1 configured to move the needle unit 17 between the first position and a second position to which the needle 17a is evacuated.

## Description

### CROSS-REFERENCE TO RELATED APPLICATION

This application claims priority to Japanese Patent Application No. 2024-057962, filed on March 29, 2024, the entire contents of which are incorporated herein by reference.

### TECHNICAL FIELD

The present disclosure relates to a cell puncture device and a microscope system.

### BACKGROUND

In conventional research and applied development related to cells, technology for puncturing cells with needles is known, in order to accurately inject chemical solutions or the like into specific cells as samples, or to suck substances inside cells. For example, Patent Literature (PTL) 1 discloses that, in a device that uses a multi-barrel nano-pipette with at least two electrodes inside multiple barrels, one barrel draws out a cell inclusion and the other injects a substance into a cell.

### CITATION LIST

### Patent Literature

PTL 1: JP 6453300 B2

### SUMMARY

In the conventional technology described in PTL 1, there is room for improvement in terms of the workability of replacing at least one of a needle for puncturing the interior of a cell or the cell to be punctured.

It would be helpful to provide a cell puncture device and a microscope system that improve the workability of replacement work required in the use of the device.

A cell puncture device according to some embodiments includes:
a needle unit having a needle configured to puncture a cell, and a needle fixer configured to fix the needle;
a needle support connected to the needle fixer, the needle support configured to arrange the needle unit at a distal end; and
a first regulator arranged with respect to the needle support, the first regulator configured to locate the needle unit in a first position in which the needle punctures the cell,
wherein the needle support includes a mover configured to move the needle unit between the first position and a second position to which the needle is evacuated.

This improves the workability of replacement work required in the use of the device. The cell puncture device can evacuate the needle unit to the second position in which the distance between the needle and the cell is greater than when the needle unit is in the first position. Therefore, when the needle unit is in the second position, the cell puncture device can provide an operator with more space between the cell and the needle, which is necessary for the replacement work.

In the cell puncture device according to one embodiment,
the needle support may have a support head configured to couple the mover and the needle fixer, and
the first regulator may be configured to locate the needle unit by contacting the support head.
Therefore, the cell puncture device can directly locate the needle unit in the first position by contacting the support head with the first regulator.

The cell puncture device according to one embodiment may further include a base arranged opposite the support head with respect to the mover, the base being configured to move the first regulator between a third position for locating the needle unit in the first position and a fourth position different from the third position. Therefore, the cell puncture device can adjust the needle at any angle using a rotation mechanism of the base.

The cell puncture device according to one embodiment may further include a second regulator attached to the base, the second regulator being configured to regulate movement of the first regulator by the base. Therefore, the cell puncture device can also adjust the rotational position of the first regulator, which is adjusted by the rotational movement of the base, to an appropriate angle for the cell to be punctured by the needle.

In the cell puncture device according to one embodiment, the first regulator may include a protrusion that protrudes from a surface of the base toward the support head and that is adjacent to the mover. Therefore, the cell puncture device can cause the first regulator to contact the support head more easily.

In the cell puncture device according to one embodiment, the first regulator may be configured to detachably fix the support head by contacting the support head. Therefore, the cell puncture device can achieve more stable positioning of the needle unit in the first position or the like using the first regulator. The cell puncture device can maintain the position of the needle unit, which has been located in the first position or the like by the first regulator, more stably using a magnetic force or the like.

In the cell puncture device according to one embodiment, at least one of the first regulator or the support head may have a magnet, and the first regulator may be configured to detachably fix the support head by a magnetic force. Therefore, the cell puncture device can maintain the position of the needle unit, which has been located in the first position or the like by the first regulator, more stably using the magnetic force. When the needle unit receives an external force greater than the magnetic force, the support head is removed from the first regulator and becomes rotatably movable. Therefore, for example, when the needle is pushed further downward than the cell, the cell puncture device causes the support head to be removed from the first regulator and rotated counterclockwise to release the force. Therefore, the cell puncture device can reduce damage to a petri dish, components of a microscope, and the like.

In the cell puncture device according to one embodiment,
the mover may be configured to rotatably move around a rotation axis, and
the first regulator may include an opposite surface that is connected to a surface of the base and that intersects a rotation direction of the mover.
Therefore, the cell puncture device can cause the opposite surface to place with respect to the support head along the rotation direction, and the opposite surface to receive the rotational movement of the support head.

In the cell puncture device according to one embodiment, the first regulator may have a positioning protrusion that protrudes so as to make point contact with the support head on the opposite surface facing the support head. This also allows the cell puncture device to achieve point contact between an end of the positioning protrusion and the support head. Therefore, the cell puncture device can improve the positioning accuracy of the support head with respect to the first regulator when the first regulator contacts the support head. As a result, the cell puncture device can also reduce a positioning error of the needle unit, which is located in the first position or the like by the first regulator.

In the cell puncture device according to one embodiment, the first regulator may be attached to the mover so as to penetrate the mover, and the first regulator may be configured to locate the needle unit by moving together with the mover. Therefore, the cell puncture device can indirectly locate the needle unit in the first position without contacting the support head with the first regulator.

The cell puncture device according to one embodiment may further include a base having an engagement structure that is arranged opposite the needle unit with respect to the mover, and with which the first regulator engages in at least one of a fifth position for locating the needle unit in the first position or a sixth position different from the fifth position.

Therefore, the cell puncture device can achieve the positioning of the needle unit by engaging the first regulator with the engagement structure of the base to stop the rotational movement of the mover. For example, the cell puncture device can locate the needle unit in the first position, in which the needle punctures the cell, by engaging the first regulator with the engagement structure of the base in the fifth position. For example, the cell puncture device can locate the needle unit in the second position, to which the needle is evacuated, by engaging the first regulator with the engagement structure of the base in the sixth position.

In the cell puncture device according to one embodiment,
an end of the first regulator may be hemispherical in shape, and
the engagement structure may include an engagement groove whose cross-sectional shape tapers in a depth direction. Therefore, the cell puncture device can also achieve two-point contact between the end of the first regulator and the engagement structure in cross-section, for example. The cell puncture device can therefore improve the positioning accuracy of the first regulator when the first regulator engages with the engagement structure. As a result, the cell puncture device can also reduce a positioning error of the needle unit, which is located by the first regulator in the first position or the like.

In the cell puncture device according to one embodiment,
the base may be configured to be rotatable so that the engagement structure rotatably moves, and
the cell puncture device may further include a second regulator that is arranged with respect to the base and that regulates rotation of the base.
Therefore, the cell puncture device can also fix the rotational position of the engagement structure, which is adjusted by the rotational movement of the base, to an appropriate angle for the cell to be punctured by the needle.

The cell puncture device according to one embodiment may further include a friction part configured to produce a frictional force against the mover when the mover moves the needle unit. Therefore, the cell puncture device allows the mover to rotate with respect to the base while the mover is receiving the frictional force from the friction part. Therefore, the cell puncture device can prevent the mover from rotating downward in the direction of gravity due to the weight of the mover itself, the weight of the needle unit, and the like, during the replacement work of the needle or the like. As a result, the operator does not need to support the mover with one hand to stop the mover from rotating while performing the replacement work, and can perform the replacement work with both hands free. This further improves the workability of the replacement work required in the use of the device.

A microscope system according to some embodiments includes:
the cell puncture device according to any one of the above, and
a microscope configured to image the cell that is to be punctured by the needle of the cell puncture device.

This improves the workability of the replacement work required in the use of the device. The microscope system can evacuate the needle unit to the second position in which the distance between the needle and the cell is greater than when the needle unit is in the first position. Therefore, when the needle unit is in the second position, the microscope system can provide the operator with more space between the cell and the needle, which is necessary for the replacement work.

According to the present disclosure, it is possible to provide the cell puncture device and the microscope system that improve the workability of the replacement work required in the use of the device.

### BRIEF DESCRIPTION OF THE DRAWINGS

In the accompanying drawings:
FIG. 1 is a schematic diagram illustrating an example configuration of a microscope system with a cell puncture device according to a first embodiment of the present disclosure;
FIG. 2A is a schematic diagram illustrating, in a front view, part of a configuration of the cell puncture device in FIG. 1;
FIG. 2B is a schematic diagram illustrating, in a top view, the configuration illustrated in FIG. 2A;
FIG. 3 is a schematic diagram illustrating an example configuration of a cell puncture device according to a second embodiment of the present disclosure;
FIG. 4 is a front view of the cell puncture device in FIG. 3;
FIG. 5 is a cross-sectional view along the line A-A in FIG. 4;
FIG. 6 is a cross-sectional view along the line B-B in FIG. 4;
FIG. 7 is a cross-sectional view, corresponding to FIG. 5, illustrating an example configuration of a variation of the cell puncture device in FIG. 3;
FIG. 8 is a cross-sectional view, corresponding to FIG. 6, illustrating an example configuration of a variation of the cell puncture device in FIG. 3;
FIG. 9 is a schematic diagram illustrating an example configuration of a cell puncture device according to a third embodiment of the present disclosure;
FIG. 10 is a cross-sectional perspective view along the line C-C in FIG. 9;
FIG. 11 is an enlarged view of an area E enclosed by a double-dotted line in FIG. 10;
FIG. 12 is a cross-sectional perspective view along the line D-D in FIG. 10; and
FIG. 13 is a cross-sectional view, corresponding to FIG. 5, along the line F-F in FIG. 9.

### DETAILED DESCRIPTION

The background and problem of conventional technology will be described in more detail.

In recent years, in research into biological systems and the like, there have been research into elucidating cell functions by injecting specific chemical solutions into cells and observing changes in the cells, and research into causing specific modifications to occur in specific cells by injecting, into the cells, chemical solutions to modify genes. In addition, there has also been applied development aiming at application to the production of chemical solutions or other substances. On the other hand, there have also been research into elucidating cell functions and applied development aiming at application to production, by sucking and recovering, from specific cells, some of components that constitute the cells. In the above research and applied development, it is required, for example, to accurately inject the chemical solutions into the specific cells or to accurately suck the components from the specific cells, so it is desired that cell puncture devices can accurately puncture cells with needles.

PTL 1 discloses conventional technology for a method and device of injecting a chemical solution into a cell by controlling the position of a fine needle using a piezoelectric element, puncturing the cell by a needle tip, and controlling voltage. Similarly, conventional technology for a method and device for sucking a substance from the interior of the cell is also disclosed.

In order to inject a chemical solution into a cell or suck a substance from a cell while minimizing damage such as a puncture to the cell punctured by the needle, it is desirable to use a needle that is as thin as possible. In recent years, it has become possible to manufacture needles with tip diameters of a few micrometers to a few nanometers by stretching glass tubes. Such glass needles are often used in cell puncture devices.

However, such thin needles can be damaged or broken by external forces. When a needle attached to a cell puncture device is damaged or broken, an operator needs to replace the needle. On the other hand, cells are often between a few micrometers and a few tens of micrometers in size. In order to accurately puncture a cell of such a size with the needle, the cell puncture device moves a needle tip close to the cell and performs position adjustment by controlling the position of the needle confirmed by an optical imaging device.

Therefore, it is not easy for the operator to replace at least one of the needle or the cell with the needle tip being located near the cell of the above size. The operator needs to temporarily evacuate the needle to another position and then replace at least one of the needle or the cell. However, when the needle is temporarily evacuated to the other position, it is not easy for the operator to accurately move the needle back to the original position. Generally, when the optical imaging device images the cell at a magnification of 10 times to 60 times, the field of view that can be imaged is limited to a few millimeters to a few hundred micrometers. Therefore, when the needle tip is not located within this field of view, the operator needs to move and find the needle. Such an operation is complicated.

In order to solve the above problem, it would be helpful to provide a cell puncture device and a microscope system that improve the workability of replacement work required in the use of the device. In the present disclosure, "the use of the device" includes, for example, the use of the cell puncture device to puncture a cell with a needle. "Replacement work" includes, for example, work to replace the needle that is attached to the cell puncture device to puncture the cell, work to replace or change the cell to be punctured, and the like.

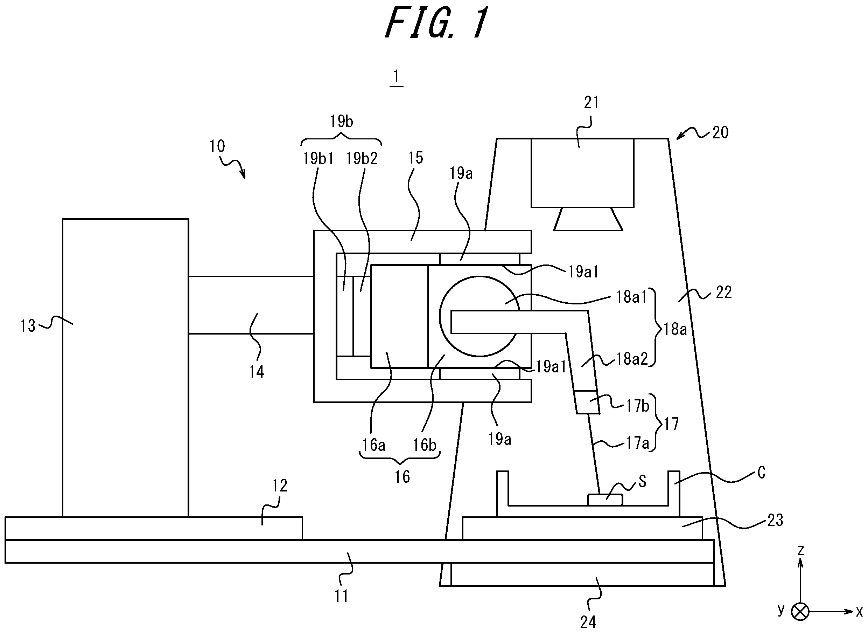

Embodiments of the present disclosure will be mainly described below with reference to the attached drawings. In the following description, x-, y-, and z-directions are with respect to the directions of the arrows in the drawings. The directions of the arrows are consistent with each other in different drawings in FIGS. 1 to 11.

### (First Embodiment)

FIG. 1 is a schematic diagram illustrating an example configuration of a microscope system 1 having a cell puncture device 10 according to a first embodiment of the present disclosure. For the sake of simplifying illustration, configurations of a first regulator 18b, a base 18c, a second regulator 18d, and the like, which are described later, of the cell puncture device 10 are omitted in FIG. 1, and part of a configuration of the cell puncture device 10 is schematically illustrated. An example of the configuration and functions of the microscope system 1 having the cell puncture device 10 according to the first embodiment will be mainly described with reference to FIG. 1. The microscope system 1 has the cell puncture device 10 and a microscope 20 that images a cell S to be punctured by a needle 17a of the cell puncture device 10.

The microscope 20 includes any microscope that can image the cell S. The microscope 20 includes, for example, a confocal microscope. The microscope 20 has any camera 21 that can image the cell S. The camera 21 constitutes an imaging unit of the microscope 20. The microscope 20 has a pillar 22 that locates the camera 21 on one side in the z-direction with respect to the cell S, so that the camera 21 can image the cell S from that side. The pillar 22 supports the camera 21 connected to an end of the pillar 22 on that side. The microscope 20 has a holder 23 that holds a petri dish C, on which the cell S is disposed, from the other side in the z-direction. The holder 23 is configured as a stage that is movable in two directions, the x- and y-directions. The microscope 20 has a support 24 that is located at an end of the pillar 22 on the other side and that supports the holder 23, which holds the petri dish C.

The cell puncture device 10 has a first fixed part 11 that is arranged with respect to the imaging unit, which images the cell S. The first fixed part 11 is, for example, fixed to the microscope 20, which images the cell S. The first fixed part 11 is configured in the shape of an arm and extends in the x-direction. The first fixed part 11 is arranged with respect to the holder 23 and the support 24 and is fixed to the microscope 20, by being screwed at one side in the x-direction onto the support 24 of the microscope 20. As an example, the first fixed part 11 is located between the holder 23 and the support 24, but is not limited to this. The holder 23 may be located under the first fixed part 11. The first fixed part 11 is not limited to being screwed onto the support 24, but may be fixed to the microscope 20 in any other manner, such as by joining, fitting, or engaging. The cell puncture device 10 can be attached to the microscope 20 via the first fixed part 11.

The cell puncture device 10 is connected to the other side of the first fixed part 11 in the x-direction, and has a pedestal 12 located on a surface of the first fixed part 11. The cell puncture device 10 is supported by the first fixed part 11 and the pedestal 12, and has a first driver 13 that protrudes from the pedestal 12 to the positive side in the z-direction. The cell puncture device 10 has an arm 14 that extends from the first driver 13 to the positive side in the x-direction. The arm 14 is arranged with respect to the first fixed part 11. For example, the arm 14 is arranged in parallel with the first fixed part 11. The first driver 13 drives the arm 14 so that the arm 14 is movable in each of the x-, y-, and z-directions with respect to the pedestal 12.

The cell puncture device 10 has a support 15 that is connected to a distal end of the arm 14 in the x-direction. The support 15 is for mounting vibration dampers 19a and a regulator 19b, which are described later. The cell puncture device 10 has a second driver 16 located inside the support 15 so as to be sandwiched by an outer frame of the support 15. Not limited to this, the second driver 16 may not be located inside the support 15. The second driver 16 may be located in another place outside the support 15, together with the vibration dampers 19a. The second driver 16 is arranged with respect to the arm 14 via the regulator 19b and the support 15. The second driver 16 is arranged with respect to the support 15 via the vibration dampers 19a.

The second driver 16 needs to move the needle 17a at a high speed in order to puncture the cell S. In order to achieve such high-speed movement of the needle 17a, the second driver 16 includes, for example, a piezoelectric element that drives the needle 17a. The second driver 16 includes a second fixed part 16a that is connected to the regulator 19b, which is described later, and a first movable part 16b that is connected to the second fixed part 16a and drives the needle 17a. The first movable part 16b is movable relative to the second fixed part 16a. The piezoelectric element contained in the first movable part 16b drives the needle 17a so that a needle tip of the needle 17a moves along the z-direction. The first movable part 16b causes the needle 17a to puncture the cell S by moving relative to the second fixed part 16a.

The cell puncture device 10 has a needle unit 17 that arranges, at a tip end, the needle 17a to puncture the cell S and that is driven by the first movable part 16b of the second driver 16. The needle unit 17 has the needle 17a that is driven by the second driver 16 and punctures the cell S, and a needle fixer 17b that fixes the needle 17a.

The needle fixer 17b allows the needle 17a to be attached to and detached from a support head 18a2, which is described later, when the needle 17a is replaced. The needle fixer 17b is detachably attached to the support head 18a2 in any manner, such as by screwing, joining, fitting, or engaging. For example, the needle fixer 17b may have a fixing screw connected to the needle 17a, and may be attached to the support head 18a2 by screwing the fixing screw into a screw arranged in the support head 18a2. The needle fixer 17b may have, for example, an axial structure compatible with the support head 18a2, and may be attached to the support head 18a2 by fitting with the support head 18a2 based on the axial structure.

The cell puncture device 10 has a needle support 18a that is connected to the needle fixer 17b and that arranges the needle unit 17 at its distal end. The needle support 18a has a mover 18a1 that moves the needle unit 17 between a first position in which the needle 17a punctures the cell S and a second position to which the needle 17a is evacuated. The needle support 18a has the support head 18a2 that couples the mover 18a1 and the needle fixer 17b. The mover 18a1 includes, as an example, a rotation mechanism to which the support head 18a2, which supports the needle unit 17 at its tip end, is connected. The mover 18a1 may undetachably or detachably fix the support head 18a2 in any manner such as by screwing, joining, fitting, or engaging.

The mover 18a1 supports the needle unit 17 rotatably clockwise and counterclockwise around a rotation axis along the y-axis. For example, the mover 18a1 defines the position of the needle unit 17 illustrated in FIG. 1, as a first position in which the needle 17a punctures the cell S. For example, the mover 18a1 defines, as a second position, any position to which the needle unit 17 is rotatably moved counterclockwise from the first position.

The cell puncture device 10 has the vibration dampers 19a that dampen vibration of the needle 17a. The vibration dampers 19a include, for example, vibration-damping rubber. The vibration dampers 19a may be arranged with respect to at least one of the arm 14 or the second driver 16. For example, in FIG. 1, the vibration dampers 19a are arranged so that vibration-damping surfaces 19a1 contact only the first movable part 16b of the second driver 16. The pair of vibration dampers 19a is arranged on both sides of the second driver 16 in the z-direction. Not limited to this, only one vibration damper 19a may be arranged with respect to the second driver 16, or three or more vibration dampers 19a may be arranged. The vibration dampers 19a are arranged between the support 15 and the second driver 16 so as to fill gaps between the support 15 and the second driver 16 along the z-direction.

The vibration dampers 19a are arranged so that the vibration-damping surfaces 19a1 intersect a puncture operation direction of the needle 17a. In the present disclosure, the "puncture operation direction" corresponds to, for example, the z-direction. For example, the vibration-damping surfaces 19a1 are orthogonal to the z-direction, which is the puncture operation direction of the needle 17a. The vibration-damping surfaces 19a1 constitute contact surfaces with the first movable part 16b in the vibration dampers 19a, and contact surfaces of the first movable part 16b in the z-direction. The vibration-damping surfaces 19a1 are included in the xy plane, as an example. When the second driver 16 vibrates and deforms the vibration dampers 19a by pressing the vibration dampers 19a, the vibration dampers 19a dampen the vibration by converting part of vibration energy of the second driver 16 into thermal energy.

The cell puncture device 10 has the regulator 19b that is arranged with respect to the second driver 16 and that regulates a movement direction of the second driver 16 to the puncture operation direction of the needle 17a. The regulator 19b includes a third fixed part 19b1 that is arranged with respect to the arm 14, and a second movable part 19b2 that is connected to the third fixed part 19b1 and the second fixed part 16a of the second driver 16. The third fixed part 19b1 is connected to, for example, an inner surface of the support 15 along the z-direction.

The regulator 19b includes, for example, a linear guide, a cross roller guide, or the like. The regulator 19b allows the second driver 16 to move in the puncture operation direction to cause the needle 17a to puncture the cell S, but regulates the movement of the second driver 16 in directions orthogonal to the puncture operation direction. For example, when the puncture operation direction is the z-direction, the regulator 19b allows the movement of the second driver 16 along the z-direction, but regulates the movement of the second driver 16 in the x- and y-directions.

The cell puncture device 10 may be configured so that the entire structure, which includes the first driver 13, the arm 14, the support 15, the second driver 16, and the needle 17a, can be evacuated from the microscope 20, by moving the pedestal 12 in the x- or y-direction relative to the first fixed part 11 or by rotatably moving the pedestal 12. The movement of the pedestal 12 in the x- or y-direction can be easily achieved by, for example, arranging a linear guide, a cross roller guide, or the like between the first fixed part 11 and the pedestal 12. The rotational movement of the pedestal 12 can be easily achieved by, for example, arranging a ball bearing, a cross roller bearing, or the like between the first fixed part 11 and the pedestal 12.

The cell puncture device 10 may further have a fixed part (not illustrated in the drawings) between the first fixed part 11 and the pedestal 12 in order to fix the relative movement of the pedestal 12 with respect to the first fixed part 11 in the x- or y-direction, or the relative rotational movement. For example, the fixed part may fix the pedestal 12 by a frictional force of a pin that can move in the z-direction and is pressed against the first fixed part 11 by a spring, or may fix the pedestal 12 by a similar pin engaging a groove provided in the first fixed part 11. The pin can be easily lifted up by arranging an operation unit to lift up the pin to the positive side in the z-direction. The pin allows the pedestal 12 to be moved and fixed relative to the first fixed part 11.

The cell puncture device 10 can accurately arrange the pedestal 12 with respect to the first fixed part 11, due to the above-described configuration. The cell puncture device 10 can reduce fluctuations in the position of the needle 17a due to play of the pedestal 12 with respect to the first fixed part 11.

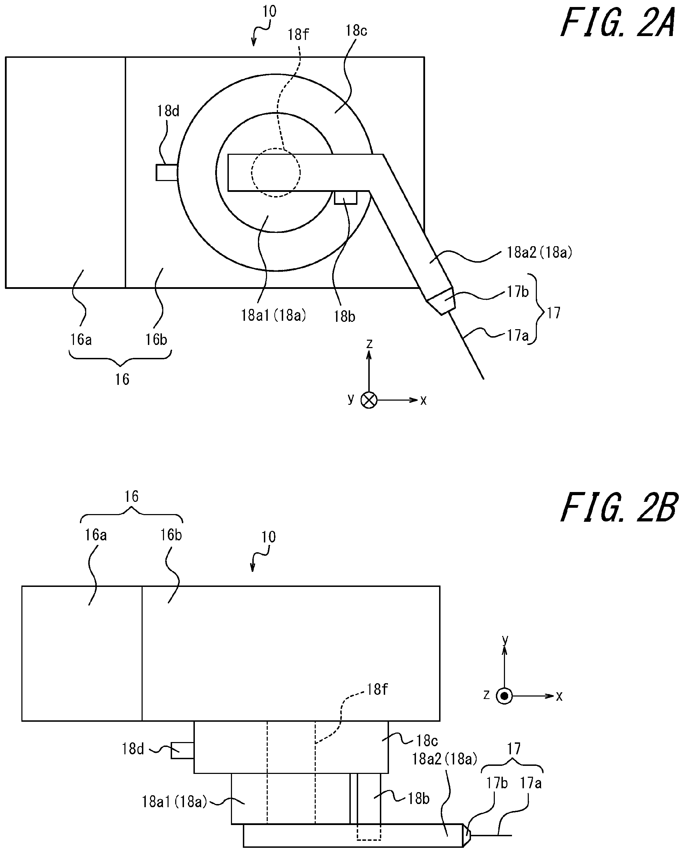

FIG. 2A is a schematic diagram illustrating, in a front view, part of a configuration of the cell puncture device 10 in FIG. 1. FIG. 2B is a schematic diagram illustrating, in a top view, the configuration illustrated in FIG. 2A. The configuration and functions related to the rotation mechanism of the mover 18a1 of the cell puncture device 10 will be mainly described with reference to FIGS. 2A and 2B.

The cell puncture device 10 further has the first regulator 18b that is adjacent to the mover 18a1 illustrated in FIG. 1 and that contacts the support head 18a2 when the needle unit 17 is in the first position. The first regulator 18b includes a protrusion that protrudes from a surface of the base 18c, which is described later, toward the support head 18a2 and that is adjacent to the mover 18a1. The first regulator 18b is in the shape of a rod and extends along the y-direction. The first regulator 18b is arranged with respect to the needle support 18a, and locates the needle unit 17 in the first position in which the needle 17a punctures the cell S. The first regulator 18b locates the needle unit 17 by contacting the support head 18a2. For example, it is assumed that the needle unit 17 rotates clockwise with respect to a rotation axis 18f of the mover 18a1. At this time, the first regulator 18b regulates further clockwise movement of the needle unit 17 by contacting the support head 18a2.

The cell puncture device 10 further has the base 18c that moves the first regulator 18b between a third position for locating the needle unit 17 in the first position, and a fourth position different from the third position. The base 18c is arranged opposite the support head 18a2 with respect to the mover 18a1. The base 18c is connected to the first regulator 18b. The base 18c determines the position of the first regulator 18b, which extends from the surface of the base 18c in the y-direction in the shape of a rod. The base 18c includes, as an example, a rotation mechanism to which the first regulator 18b is connected. The base 18c supports the first regulator 18b rotatably clockwise and counterclockwise around the rotation axis 18f along the y-axis. For example, the base 18c determines, as the third position, the position of the first regulator 18b as illustrated in FIGS. 2A and 2B when the needle unit 17 is located in the first position. For example, the base 18c determines, as a fourth position, any position to which the first regulator 18b has rotatably moved from the third position.

The base 18c is connected to the first movable part 16b of the second driver 16. The base 18c is rotatably movable relative to the first movable part 16b. In addition, the mover 18a1 is connected to the base 18c. The mover 18a1 is rotatably movable relative to the base 18c. Each of the base 18c and the mover 18a1 is rotatably movable clockwise and counterclockwise around the rotation axis 18f, which is common to each other. In FIG. 2A, the rotation axis 18f is illustrated as being common to the base 18c and the mover 18a1, but is not limited to this. The rotation axis 18f does not have to be common to the base 18c and the mover 18a1. Each of the base 18c and the mover 18a1 may have a different rotation axis 18f from each other.

The cell puncture device 10 further has the second regulator 18d that is attached to the base 18c and that regulates the movement of the first regulator 18b by the base 18c. The second regulator 18d includes, for example, a knurled screw as a screw-type knob. The second regulator 18d fixes the rotational position of the base 18c to a predetermined relative angle with respect to the first movable part 16b of the second driver 16. For example, the knurled screw of the second regulator 18d is attached to the base 18c, and fixes the base 18c by being pressed against the rotation axis 18f.

At this time, the first regulator 18b is fixed at a predetermined relative angle with respect to the mover 18a1, in conjunction with the fixing of the base 18c. As described above, the first regulator 18b, which extends from the base 18c, locates the needle unit 17 in the first position at a predetermined angle with respect to a contact surface of the support head 18a2 with the first regulator 18b, by contacting the support head 18a2 with being fixed in the third position.

The cell puncture device 10 according to the first embodiment, as described above, improves the workability of replacement work required in the use of the device. The cell puncture device 10 has the first regulator 18b that locates the needle unit 17 in the first position in which the needle 17a punctures the cell S, and the mover 18a1 that moves the needle unit 17 between the first position and the second position to which the needle 17a is evacuated. The cell puncture device 10 can thereby evacuate the needle unit 17 to the second position in which a clearance distance between the needle 17a and the cell S is greater than when the needle unit 17 is in the first position. Therefore, when the needle unit 17 is in the second position, the cell puncture device 10 can provide an operator with more space between the cell S and the needle 17a, which is necessary for the replacement work.

For example, the operator can easily replace the needle 17a, which penetrates a cell wall and punctures the interior of the cell S, in wide space. In addition, the operator can easily return a replaced needle 17a to the original position near the cell S, because the first position is determined by the first regulator 18b of the cell puncture device 10. Besides, the operator can also easily change the cell S to be punctured, instead of or in addition to the replacement of the needle 17a. For example, the operator can easily evacuate the needle unit 17 to the second position when replacing the petri dish C, and then relocate the needle 17a within the original field of view of the microscope 20 after replacing the petri dish C.

For example, the cell puncture device 10 can rotate the needle unit 17 at the distal end of the support head 18a2 connected to the mover 18a1, by rotating the mover 18a1 relative to the base 18c. Therefore, even when the cell puncture device 10 is mounted on the microscope 20, as illustrated in FIG. 1, it is possible to move the needle 17a away from the cell S and the petri dish C containing the cell S by rotational movement. The operator can easily replace at least one of the needle 17a or the cell S by such rotational movement of the needle unit 17. After the replacement work, the operator can locate, using the first regulator 18b, the support head 18a2 at a predetermined angle corresponding to the first position of the needle unit 17. The operator can also locate the needle tip of the needle 17a near the cell S and within the field of view of the microscope 20 even after the replacement work. This improves the workability of the replacement work required in the use of the device.

The first regulator 18b locates the needle unit 17 by contacting the support head 18a2. Therefore, the cell puncture device 10 can directly locate the needle unit 17 in the first position by contacting the support head 18a2 with the first regulator 18b.

The cell puncture device 10 further has the base 18c that moves the first regulator 18b between the third position for locating the needle unit 17 in the first position, and the fourth position different from the third position. This allows the cell puncture device 10 to adjust the needle 17a to any angle, using the rotation mechanism of the base 18c.

Assuming that the needle 17a is located in parallel with the puncture operation direction, in contrast to FIG. 1 and the like, the needle 17a punctures the cell S straight. At this time, the damage to the cell S is minimal. However, in this case, in the microscope 20 as an inverted microscope, for example, the needle 17a is arranged in parallel with the optical axis of the microscope 20, and the puncture operation direction is also in parallel with the optical axis. Therefore, the support head 18a2 may block the field of view of the microscope 20 and prevent the operator from seeing the position of the needle tip of the needle 17a.

Therefore, when the operator sees the position of the needle tip of the needle 17a and adjusts the position of the needle tip to perform a puncture using the microscope 20, the needle 17a needs to be tilted at a slight angle to at least one of the optical axis of the microscope 20 or the puncture operation direction, as illustrated in FIG. 1. When this angle is large, it is easier for the operator to see the position of the needle tip of the needle 17a, but damage to the cell S is large because the needle 17a punctures the cell S at a tilt. Therefore, it is desirable to be able to change this angle according to the cell S to be punctured by the needle 17a.

The cell puncture device 10 can adjust, using the base 18c, the rotational position of the first regulator 18b. Therefore, the cell puncture device 10 can also adjust, using the rotation mechanism of the base 18c, the rotational position of the first regulator 18b to an appropriate angle for the cell S to be punctured by the needle 17a.

The cell puncture device 10 further has the second regulator 18d that is attached to the base 18c and that regulates the movement of the first regulator 18b by the base 18c. The cell puncture device 10 can thereby fix the rotational position of the first regulator 18b, which is adjusted by the rotational movement of the base 18c, to an appropriate angle for the cell S to be punctured by the needle 17a.

The first regulator 18b includes the protrusion that extends from the surface of the base 18c towards the support head 18a2 and that is adjacent to the mover 18a1. Therefore, the cell puncture device 10 can make the first regulator 18b contact with the support head 18a2 more easily.

In the first embodiment described above, for example, the first fixed part 11 is described as being fixed to the microscope 20 that images the cell S, but is not limited to this. The first fixed part 11 may be attached to the microscope 20 in another aspect, as long as the first fixed part 11 is arranged with respect to the imaging unit that images the cell S. For example, the first fixed part 11 may be incorporated inside the microscope 20, instead of being fixed so as to extend outside the microscope 20 as described above.

In the first embodiment described above, the cell puncture device 10 is described as further having the base 18c that moves the first regulator 18b between the third position for locating the needle unit 17 in the first position and the fourth position different from the third position, but is not limited to this. The cell puncture device 10 may not have the base 18c. In the cell puncture device 10, the first regulator 18b may not be movable by a component such as the base 18c, and may be fixed in a predetermined fixed position.

In the first embodiment described above, the cell puncture device 10 is described as further having the second regulator 18d that is attached to the base 18c and that regulates the movement of the first regulator 18b by the base 18c, but is not limited to this. The cell puncture device 10 may not have the second regulator 18d.

In the first embodiment described above, the mover 18a1 is described as including the rotation mechanism, but is not limited to this. The mover 18a1 may include a translation mechanism. The mover 18a1 may achieve the movement of the needle unit 17 between the first position in which the needle 17a punctures the cell S and the second position to which the needle 17a is evacuated, based on translation movement in addition to or instead of rotational movement.

In the first embodiment described above, the base 18c is described as including the rotation mechanism, but is not limited to this. The base 18c may include a translation mechanism. The base 18c may achieve the movement of the first regulator 18b between the third position for locating the needle unit 17 in the first position and the fourth position different from the third position, based on translation movement in addition to or instead of rotational movement.

In the first embodiment described above, the cell puncture device 10 may further have a friction part 18e that produces a frictional force against the mover 18a1 when the mover 18a1 moves the needle unit 17. For example, the friction part 18e may include a component such as a spring washer, a conical spring, or a wave washer arranged between the mover 18a1 and the base 18c around the rotation axis 18f in FIG. 2B. When the mover 18a1 rotates with respect to the base 18c, the component rubs against the mover 18a1 and directly produces a frictional force against the mover 18a1.

The provision of the friction part 18e in the cell puncture device 10 makes it possible for the mover 18a1 to receive the frictional force from the friction part 18e while the mover 18a1 is rotating with respect to the base 18c. Therefore, the cell puncture device 10 can prevent the mover 18a1 from rotating downward in the direction of gravity, due to the weight of the mover 18a1 itself, the weight of the needle unit 17, and the like, during the replacement work of the needle 17a or the like. As a result, the operator does not need to support the mover 18a1 with one hand to stop the mover 18a1 from rotating while performing the replacement work, and can perform the replacement work with both hands free. This further improves the workability of the replacement work required in the use of the device.

**In** the first embodiment described above, even in a case in which one component is directly connected to another component, the present disclosure is not limited to such a configuration relationship. Yet another component may be interposed between the one component and the other component, or the one component and the other component may be indirectly connected to each other, as long as the functions of the cell puncture device 10 according to the first embodiment can be achieved.

**In** the first embodiment described above, the second driver 16 is divided into two components, i.e., the second fixed part 16a and the first movable part 16b, along the x-direction, but is not limited to this. The second driver 16 may be divided into two components, i.e., the second fixed part 16a and the first movable part 16b, along another direction different from the x-direction. For example, the second driver 16 may be divided into two components, i.e., the second fixed part 16a and the first movable part 16b, along the y-direction. In other words, the second fixed part 16a and the first movable part 16b may be arranged not along the x-direction but along the y-direction.

**In** the first embodiment described above, the regulator 19b is divided into two components, i.e., the third fixed part 19b1 and the second movable part 19b2, along the x-direction, but is not limited to this. The regulator 19b may be divided into two components, i.e., the third fixed part 19b1 and the second movable part 19b2, along another direction different from the x-direction. For example, the regulator 19b may be divided into two components, i.e., the third fixed part 19b1 and the second movable part 19b2, along the y-direction. In other words, the third fixed part 19b1 and the second movable part 19b2 may be arranged not along the x-direction but along the y-direction.

In the first embodiment described above, the first regulator 18b locates the needle unit 17 by contacting the support head 18a2. For example, the first regulator 18b contacts the support head 18a2 due to the weight of the support head 18a2 based on gravity. However, the first regulator 18b may achieve interaction based on a magnetic force or the like between the first regulator 18b and the support head 18a2, as in a configuration of a third embodiment described later. The cell puncture device 10 can thereby maintain contact between the first regulator 18b and the support head 18a2 more firmly when the needle 17a punctures the cell S.

In addition, when the needle unit 17 is subjected to an external force greater than the above-described magnetic force, the support head 18a2 is removed from the first regulator 18b and rotatably movable. Therefore, in the cell puncture device 10, for example, when the needle 17a is pushed further downward than the cell S, the support head 18a2 is removed from the first regulator 18b and rotated counterclockwise to release the force. Therefore, the cell puncture device 10 can reduce damage to the petri dish C, components of the microscope 20, and the like.

### (Second Embodiment)

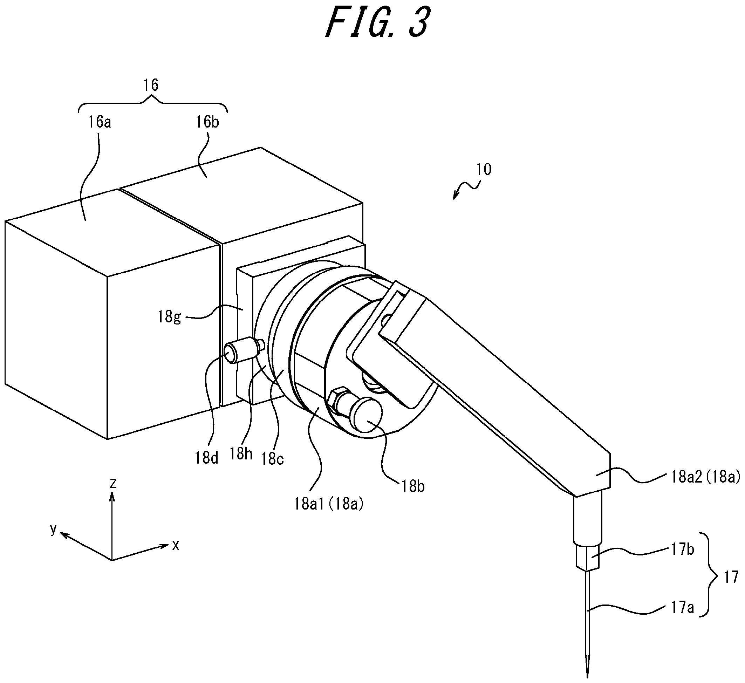

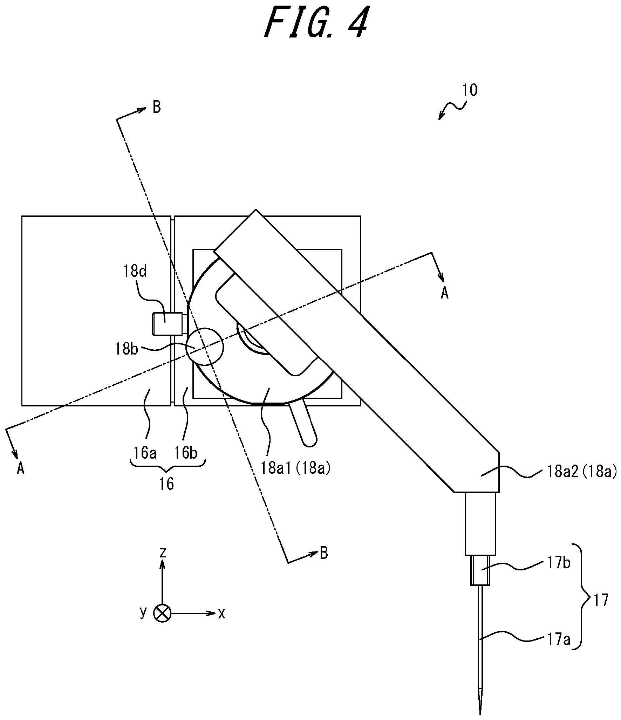

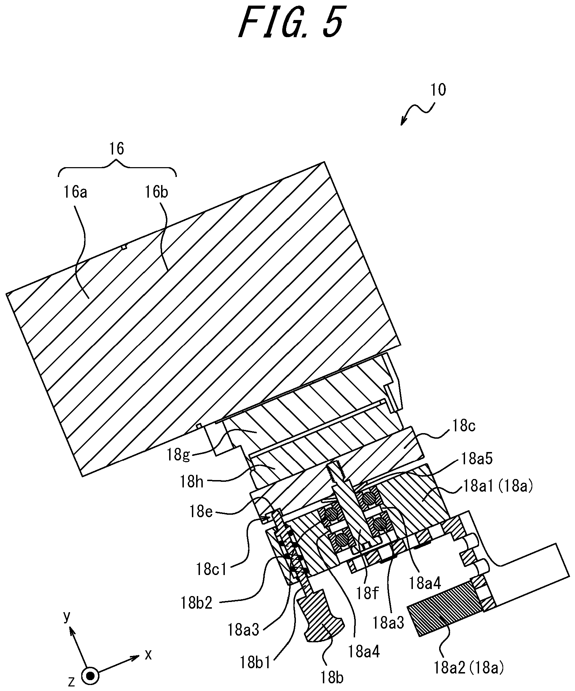

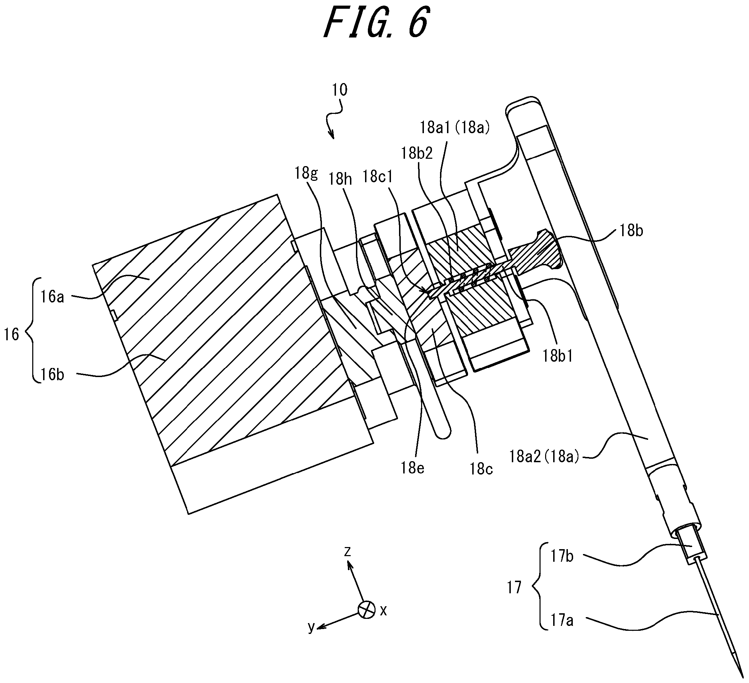

FIG. 3 is a schematic diagram illustrating an example configuration of a cell puncture device 10 according to a second embodiment of the present disclosure. In FIG. 3, only part of the configuration of the cell puncture device 10 is schematically illustrated for the sake of simplifying illustration. FIG. 4 is a front view of the cell puncture device 10 in FIG. 3. FIG. 5 is a cross-sectional view along the line A-A in FIG. 4. FIG. 6 is a cross-sectional view along the line B-B in FIG. 4. An example of the configuration and functions of the cell puncture device 10 according to the second embodiment will be mainly described with reference to FIGS. 3 to 6.

In the first embodiment described above, the first regulator 18b is described as locating the needle unit 17 by contacting the support head 18a2, but is not limited to this. The first regulator 18b may locate the needle unit 17 without contacting the support head 18a2. The cell puncture device 10 according to the second embodiment differs from the first embodiment in this point. The other components, functions, effects, variations, and the like are the same as those in the first embodiment, and the corresponding descriptions also apply to the cell puncture device 10 according to the second embodiment. In the following, the same components as in the first embodiment are indicated with the same reference numerals, and descriptions thereof are omitted. The point that differs from the first embodiment will be mainly described.

In the second embodiment, as mainly illustrated in FIGS. 5 and 6, a first regulator 18b may be attached to a mover 18a1 so as to penetrate the mover 18a1, and may locate a needle unit 17 by moving together with the mover 18a1. The first regulator 18b may include a pin 18b1 that extends in the y-direction. In the first regulator 18b, a spring 18b2, which biases the pin 18b1, may be arranged around the pin 18b1. The spring 18b2 pushes the pin 18b1 toward a base 18c. The base 18c may have an engagement structure 18c1 with which the pin 18b1 of the first regulator 18b engages. The engagement structure 18c1 may include an engagement groove or an engagement hole.

The base 18c is arranged opposite the needle unit 17 with respect to the mover 18a1. The base 18c may have the engagement structure 18c1 with which the first regulator 18b engages in at least one of a fifth position for locating the needle unit 17 in a first position or a sixth position different from the fifth position. FIGS. 5 and 6 illustrate the engagement structure 18c1 when the first regulator 18b is in the fifth position. Instead of or in addition to this, the base 18c may have another engagement structure 18c1 in a different position from the engagement structure 18c1 illustrated in FIGS. 5 and 6, with the same shape as or a different shape from the engagement structure 18c1 illustrated in FIGS. 5 and 6. The first regulator 18b engages with the other engagement structure 18c1 when being in the sixth position. The sixth position may be, for example, the position of the first regulator 18b for locating the needle unit 17 in the second position to which the needle 17a is evacuated. For example, when the engagement structure 18c1 has a different shape, the engagement structure 18c1 in the sixth position for evacuating the needle 17a does not need to have as high positional accuracy as in the fifth position, which corresponds to the position of the needle 17a for performing a puncture, and may have a larger groove width or hole width.

In a case in which the pin 18b1 of the first regulator 18b is biased by the spring 18b2, the spring 18b2 is stretched when the first regulator 18b engages with the engagement structure 18c1 of the base 18c. On the other hand, when the mover 18a1 rotatably moves with respect to the base 18c and the first regulator 18b is disengaged from the engagement structure 18c1 of the base 18c, an end of the pin 18b1 comes into contact with a surface of the base 18c, which does not have the engagement structure 18c1, and the spring 18b2 is slightly compressed. At this time, the end of the first regulator 18b is pressed against the surface by a restoring force of the spring 18b2 itself. When the mover 18a1 continues rotational movement, the end of the first regulator 18b slides while being pressed against the surface of the base 18c.

For example, when the first regulator 18b is pulled, the end of the first regulator 18b can be disengaged from the engagement structure 18c1 and come out into an area of the base 18c in which no engagement structure 18c1 is present. When the pull of the first regulator 18b is stopped in that state, the spring 18b2 presses the end of the first regulator 18b against the base 18c, and a frictional force is produced.

Therefore, in the cell puncture device 10 according to the second embodiment, a friction part 18e, which produces a frictional force against the mover 18a1 when the mover 18a1 moves the needle unit 17, may include the end of the first regulator 18b. When the mover 18a1 rotatably moves with respect to the base 18c, the end of the first regulator 18b slides on the surface of the base 18c and indirectly produces the frictional force against the mover 18a1.

As illustrated in FIGS. 3 and 4, the base 18c is configured to be rotatable so that the engagement structure 18c1 can rotatably move relative to a first movable part 16b. In this case, a second regulator 18d is arranged with respect to the base 18c and regulates the rotation of the base 18c with respect to the first movable part 16b. The second regulator 18d may be attached to a rotation stage located opposite the mover 18a1 with respect to the base 18c. The rotation stage may be constituted of a fourth fixed part 18g connected to the first movable part 16b, and a third movable part 18h attached to the mover 18a1's side with respect to the fourth fixed part 18g. The base 18c and the third movable part 18h may be fixed to each other by screwing using a screw or the like. The base 18c and the third movable part 18h may move integrally with each other. The fourth fixed part 18g may be arranged further on the first movable part 16b's side than the third movable part 18h. The third movable part 18h and the fourth fixed part 18g may be connected to each other by a rotation axis or the like. The third movable part 18h may be rotatably movable relative to the fourth fixed part 18g.

As illustrated in FIG. 5, the mover 18a1 has a rotation mechanism arranged around a rotation axis 18f. The rotation mechanism includes, for example, bearings 18a3 and a bearing support 18a4. The mover 18a1 has a biasing mechanism that biases one ends of the bearings 18a3 toward the negative side in the y-direction. The biasing mechanism includes, for example, a Belleville spring 18a5. The bearing support 18a4 may be arranged as a protrusion in a cross-sectional view formed on an inner wall of the mover 18a1 between the two bearings 18a3 arranged along the y-direction. Not limited to this, the bearing support 18a4 may be constituted of a part that is in the shape of a ring and that is different from the mover 18a1. The bearing support 18a4 receives a thrust load from the two bearings 18a3. The Belleville spring 18a5 fixes the positions of the bearings 18a3 by pushing the inside of one end of the bearing 18a3. FIG. 5 illustrates ball bearings, and since the two bearings 18a3 are used in combination, the use of angular contact ball bearings makes it possible to receive a stronger force in the thrust direction.

The cell puncture device 10 according to the second embodiment as described above can obtain the same effects as the first embodiment. In addition, in the cell puncture device 10, the first regulator 18b is attached to the mover 18a1 so as to penetrate the mover 18a1, and moves together with the mover 18a1 to locate the needle unit 17, which allows the needle unit 17 to be indirectly located in the first position, with the support head 18a2 not contacting the first regulator 18b.

The base 18c of the cell puncture device 10 has the engagement structure 18c1 with which the first regulator 18b engages in at least one of the fifth position for locating the needle unit 17 in the first position or the sixth position different from the fifth position. The cell puncture device 10 can thereby locate the needle unit 17 by engaging the first regulator 18b with the engagement structure 18c1 of the base 18c to stop the rotational movement of the mover 18a1. For example, the cell puncture device 10 can locate the needle unit 17 in the first position in which the needle 17a punctures the cell S, by engaging the first regulator 18b with the engagement structure 18c1 of the base 18c in the fifth position. For example, the cell puncture device 10 can locate the needle unit 17 in the second position to which the needle 17a is evacuated, by engaging the first regulator 18b with the engagement structure 18c1 of the base 18c in the sixth position.

The cell puncture device 10 further has the second regulator 18d that is arranged with respect to the base 18c, which is configured rotatable so that the engagement structure 18c1 rotatably moves, and that regulates the rotation of the base 18c. Therefore, the cell puncture device 10 can fix the rotational position of the engagement structure 18c1, which is adjusted by the rotational movement of the base 18c, to an appropriate angle for the cell S to be punctured by the needle 17a.

The cell puncture device 10 has the friction part 18e, which allows the mover 18a1 to rotate with respect to the base 18c while the mover 18a1 is indirectly receiving the frictional force via the friction part 18e. Therefore, the cell puncture device 10 can prevent the mover 18a1 from rotating downward in the direction of gravity due to the weight of the mover 18a1 itself, the weight of the needle unit 17, and the like, while the needle 17a or the like is being replaced. As a result, an operator does not need to support the mover 18a1 with one hand to stop the rotation of the mover 18a1 while performing replacement work, and can perform the replacement work with both hands free. This further improves the workability of the replacement work required in the use of the device.

In the second embodiment described above, the base 18c is described as having the one or two engagement structures 18c1, but is not limited to this. The base 18c may have three or more engagement structures 18c1.

In the second embodiment described above, the cell puncture device 10 is described as further having the second regulator 18d that is arranged with respect to the base 18c and that regulates the rotation of the base 18c, but is not limited to this. The cell puncture device 10 may not have the second regulator 18d.

In the second embodiment described above, the cell puncture device 10 is described as further having the friction part 18e that produces the frictional force against the mover 18a1 when the mover 18a1 moves the needle unit 17, but is not limited to this. The cell puncture device 10 may not have the friction part 18e. In other words, when the mover 18a1 rotatably moves with respect to the base 18c, the end of the first regulator 18b does not slide on the surface of the base 18c and does not produce the frictional force against the mover 18a1.

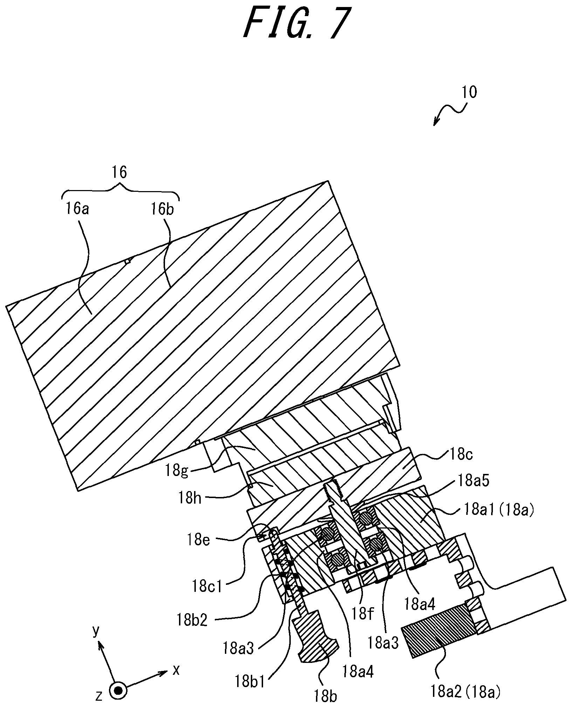

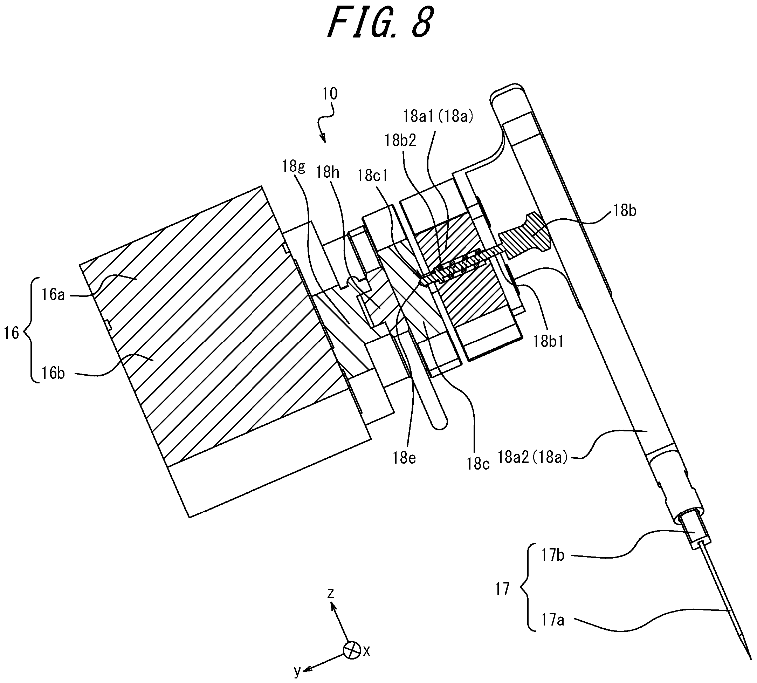

FIG. 7 is a cross-sectional view, corresponding to FIG. 5, illustrating an example configuration of a variation of the cell puncture device 10 in FIG. 3. FIG. 8 is a cross-sectional view, corresponding to FIG. 6, illustrating the example configuration of the variation of the cell puncture device 10 in FIG. 3. In the second embodiment described above, the engagement groove in the engagement structure 18c1 of the base 18c is configured to be approximately rectangular in the cross-sectional shape, but is not limited to this. The engagement structure 18c1 of the base 18c may include an engagement groove whose cross-sectional shape tapers in a depth direction. For example, as illustrated in FIG. 8, the engagement groove may have a cross-sectional shape of the letter V whose width narrows from the surface of the base 18c inwardly in the depth direction.

The end of the first regulator 18b may be hemispherical in shape. When the first regulator 18b engages with the engagement structure 18c1 of the base 18c, the hemispherical end of the first regulator 18b may contact the pair of inclined surfaces of the V-shaped engagement groove in the engagement structure 18c1. The pair of inclined surfaces of the V-shaped engagement groove may contact a circumferential portion of the hemispherical end of the first regulator 18b from both sides.

The cell puncture device 10 can also achieve the above-described two-point contact between the end of the first regulator 18b and the engagement structure 18c1 in the cross-section, for example, due to the engagement structure 18c1 including the engagement groove whose cross-sectional shape tapers in the depth direction. Therefore, the cell puncture device 10 can improve the positioning accuracy of the first regulator 18b when the first regulator 18b engages with the engagement structure 18c1. As a result, the cell puncture device 10 can also reduce a positioning error of the needle unit 17, which is located by the first regulator 18b in the first position or the like.

### (Third Embodiment)

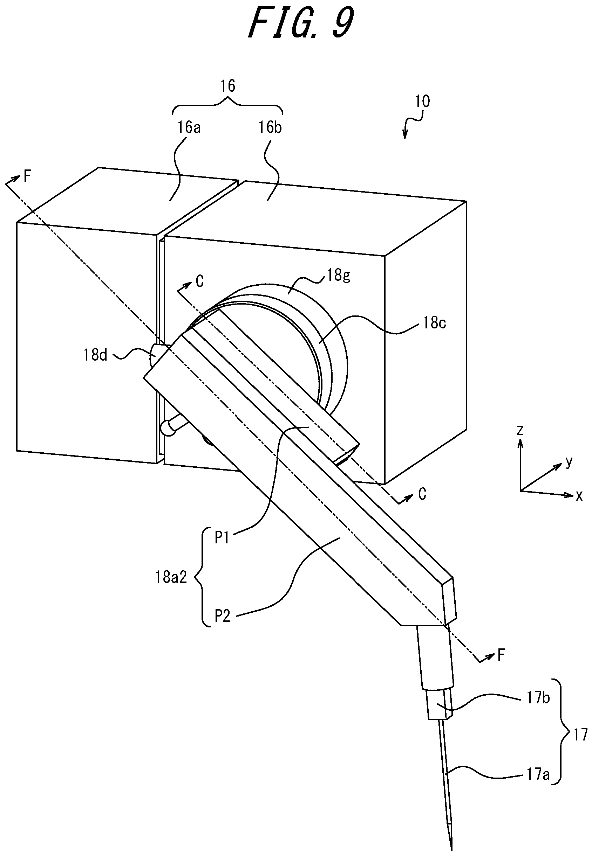

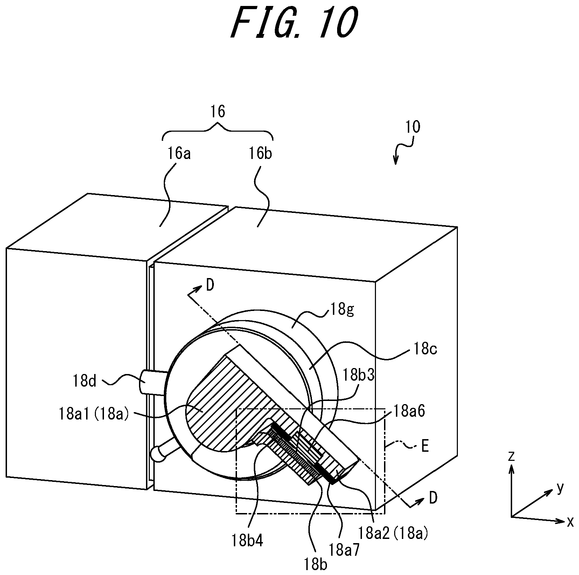

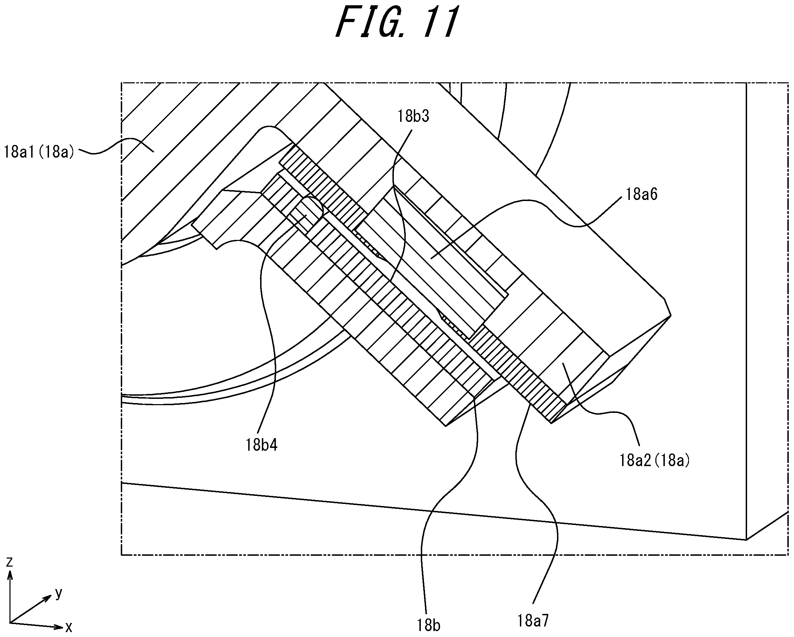

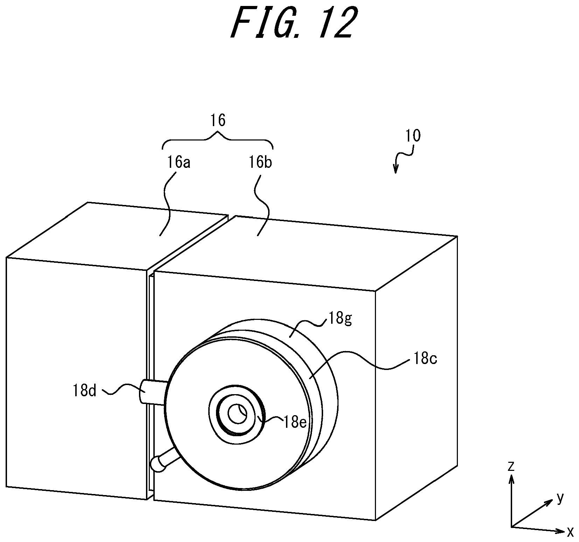

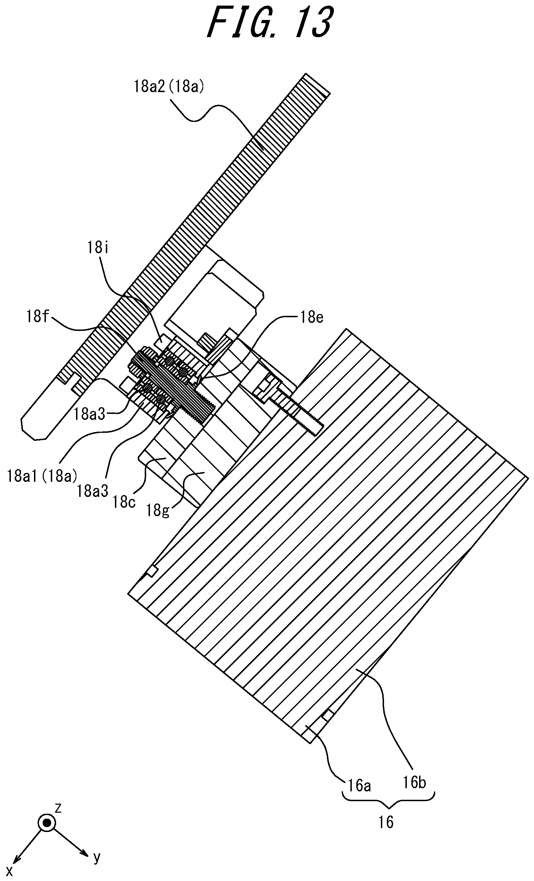

FIG. 9 is a schematic diagram illustrating an example configuration of a cell puncture device 10 according to a third embodiment of the present disclosure. In FIG. 9, only part of the configuration of the cell puncture device 10 is schematically illustrated for the sake of simplifying illustration. FIG. 10 is a cross-sectional perspective view along the line C-C in FIG. 9. FIG. 11 is an enlarged view of an area E enclosed by a double-dotted line in FIG. 10. FIG. 12 is a cross-sectional perspective view along the line D-D in FIG. 10. FIG. 13 is a cross-sectional view, corresponding to FIG. 5, along the line F-F in FIG. 9. An example of the configuration and functions of the cell puncture device 10 according to the third embodiment will be mainly described with reference to FIGS. 9 to 13.

As illustrated in FIG. 9, a support head 18a2 is configured as two components connected to each other. For example, as illustrated in FIG. 9, configuring the support head 18a2 as the two components makes it possible to change the position of a second part P2 of the support head 18a2 on the front side, relative to a first part P1 of the support head 18a2 on the back side, which is the positive side of the y-axis. For example, the second part P2 can be moved using a screw and a long hole so that the position of a needle 17a can be moved close to or away from a rotation center of the support head 18a2. In addition, the second part P2 can be removed from the first part P1.

In the cell puncture device 10 according to the third embodiment, as in the first embodiment, a first regulator 18b locates a needle unit 17 by contacting the support head 18a2. The first regulator 18b detachably fixes the support head 18a2 by contacting the support head 18a2. The other configurations, functions, effects, variations, and the like are the same as those in the first embodiment, and the corresponding descriptions also apply to the cell puncture device 10 according to the third embodiment. In the following, the same components as in the first embodiment are indicated with the same reference numerals, and descriptions thereof are omitted. The points that differ from the first embodiment will be mainly described.

In the cell puncture device 10 according to the third embodiment, at least one of the first regulator 18b or the support head 18a2 may have a magnet. For example, as illustrated in FIGS. 10 and 11, a support head 18a2 of the cell puncture device 10 may have a magnet 18a6. Part of the magnet 18a6 may be exposed to a first regulator 18b on a first opposite surface 18a7 of the support head 18a2, which faces the first regulator 18b. Not limited to this, the magnet 18a6 may not be exposed on the first opposite surface 18a7 by using nonmagnetic stainless steel such as austenitic stainless steel represented by SUS304 or the like. Even in such a case, the magnet 18a6 can attract a second opposite surface 18b3, which is described later. The magnet 18a6 may magnetically interact with the first regulator 18b, as a magnetic substance, for example. The first regulator 18b may detachably fix the support head 18a2 by a magnetic force.

At this time, the first regulator 18b may detachably fix the support head 18a2 so that a second regulation force, which regulates the movement of the support head 18a2 upward in the direction of gravity, is weaker than a first regulation force, which regulates the movement of the support head 18a2 downward in the direction of gravity. In other words, the first regulator 18b may strongly regulate the movement of the support head 18a2 downward in the direction of gravity, e.g., to the negative side in the z-direction, but may weakly regulate the movement of the support head 18a2 upward in the direction of gravity, e.g., to the positive side in the z-direction. Upon receiving an external force greater than the magnetic force acting between the first regulator 18b and the support head 18a2, the support head 18a2 may be removed from the first regulator 18b and rotatably moved counterclockwise.

As in the first and second embodiments, a mover 18a1 rotates around a rotation axis 18f illustrated in FIG. 13. However, in contrast to the second embodiment, a bearing support 18a4 is not arranged. A Belleville spring 18a5 is not arranged either. Two bearings 18a3 are press-fitted along the y-direction. The outside of the bearings 18a3 and the mover 18a1 are integrated with each other by press-fitting. The inside of the bearings 18a3 and the rotation axis 18f are integrated with each other by press-fitting. The mover 18a1 rotates by rotating the inside and outside of the bearings 18a3. Even in the case of press-fitting as described above, bearing pullout prevention screws 18i may be provided.

The bearings 18a3 may be installed not by press-fitting but by clearance fitting. In the case of clearance fitting, the bearings 18a3 may come out of the mover 18a1, so the bearing pullout prevention screws 18i are used to prevent the bearings 18a3 from coming out of the mover 18a1.

In both of the mover 18a1 and outer rings of the bearings 18a3, and the rotation axis 18f and inner rings of the bearings 18a3, adopting press-fitting eliminates gaps between the cylinder and the bearings 18a3, thus allowing rotation without play. On the other hand, clearance fitting eases assembly work in attaching the bearings 18a3 to the mover 18a1. As described above, the mover 18a1 can rotate around the rotation axis 18f using the bearings 18a3.

The mover 18a1 has a rotation mechanism arranged around the rotation axis 18f. The rotation mechanism includes, for example, the bearings 18a3. At this time, as illustrated in FIGS. 10 and 11, the first regulator 18b includes the second opposite surface 18b3, which is connected to a surface of a base 18c and faces the support head 18a2.

The second opposite surface 18b3 may be formed of a material that has a magnetic substance so as to be attracted by a magnetic force. For example, the material may be a metal such as iron, nickel, or cobalt, an alloy thereof, or a ferrite-based stainless steel such as SUS430 or SUS440C, or a martensitic stainless steel.

The first regulator 18b may have a positioning protrusion 18b4 that protrudes to make point contact with the support head 18a2, on the second opposite surface 18b3, which faces the support head 18a2. An end of the positioning protrusion 18b4 may be hemispherical. The hemispherical end of the positioning protrusion 18b4 may protrude from the second opposite surface 18b3 towards the support head 18a2.

As illustrated in FIGS. 12 and 13, the cell puncture device 10 may further have a friction part 18e that produces a frictional force against the mover 18a1 when the mover 18a1 moves the needle unit 17, as in the first embodiment. For example, the friction part 18e may include a component such as a spring washer, a conical spring, or a wave washer, which is arranged between the mover 18a1 and the base 18c in FIG. 12. When the mover 18a1 rotates with respect to the base 18c, the component rubs against the mover 18a1 and directly produces a frictional force against the mover 18a1.

In the cell puncture device 10, as in the second embodiment, the base 18c, a second regulator 18d, and a fourth fixed part 18g, and the like may be arranged on a first movable part 16b's side with respect to the mover 18a1. As in the second embodiment, a third movable part 18h may be further arranged between the base 18c and the fourth fixed part 18g.

The cell puncture device 10 according to the third embodiment, as described above, can obtain the same effects as in the first embodiment. In addition, the cell puncture device 10 can achieve more stable positioning of the needle unit 17 in the first position or the like, by the first regulator 18b contacting the support head 18a2 and detachably fixing the support head 18a2. The cell puncture device 10 can more stably maintain, using the magnetic force or the like, the position of the needle unit 17, which is located in the first position or the like by the first regulator 18b.

Due to the first regulator 18b detachably fixing the support head 18a2 by the magnetic force, the cell puncture device 10 can more stably maintain, using the magnetic force, the position of the needle unit 17 that is located in the first position or the like by the first regulator 18b. When the needle unit 17 receives an external force greater than the magnetic force, the support head 18a2 is removed from the first regulator 18b and becomes rotatably movable. Therefore, for example, when the needle 17a is pushed further downward than a cell S, the cell puncture device 10 causes the support head 18a2 to be removed from the first regulator 18b and rotated counterclockwise to release the force. Therefore, the cell puncture device 10 can reduce damage to a petri dish C, components of a microscope 20, and the like.

The first regulator 18b includes the second opposite surface 18b3 that is connected to the surface of the base 18c and that intersects the rotation direction of the mover 18a1. Therefore, the cell puncture device 10 can cause the second opposite surface 18b3 to place with respect to the support head 18a2 along the rotation direction, and the second opposite surface 18b3 to receive the rotational movement of the support head 18a2.

The first regulator 18b has the positioning protrusion 18b4 that protrudes to make point contact with the support head 18a2 on the second opposite surface 18b3, which faces the support head 18a2. This allows the cell puncture device 10 to achieve point contact between the end of the positioning protrusion 18b4 and the support head 18a2. Therefore, the cell puncture device 10 can improve the positioning accuracy of the support head 18a2 with respect to the first regulator 18b when the first regulator 18b contacts the support head 18a2. As a result, the cell puncture device 10 can also reduce a positioning error of the needle unit 17, which is located in the first position or the like by the first regulator 18b. Note that, the fourth fixed part 18g and the base 18c may constitute a rotation stage. As illustrated in FIG. 10, the second regulator 18d is arranged in the fourth fixed part 18g, and regulates the rotation of the base 18c with respect to the first movable part 16b. The above-described rotation stage may be constituted of the fourth fixed part 18g, which is connected to the first movable part 16b, and the base 18c as a movable part attached to the mover 18a1's side with respect to the fourth fixed part 18g. The fourth fixed part 18g may be arranged further on the first movable part 16b's side than the base 18c. The base 18c and the fourth fixed part 18g may be connected to each other by a rotation axis or the like. The base 18c may be rotatably movable relative to the fourth fixed part 18g.

The provision of the friction part 18e in the cell puncture device 10 makes it possible for the mover 18a1 to receive the frictional force from the friction part 18e while the mover 18a1 is rotating with respect to the base 18c. Therefore, the cell puncture device 10 can prevent the mover 18a1 from rotating downward in the direction of gravity due to the weight of the mover 18a1 itself, the weight of the needle unit 17, and the like, during replacement work of the needle 17a or the like. As a result, an operator does not need to support the mover 18a1 with one hand to stop the mover 18a1 from rotating while performing the replacement work, and can perform the replacement work with both hands free. This further improves the workability of the replacement work required in the use of the device.

In the third embodiment described above, the cell puncture device 10 is described as further having the friction part 18e that produces the frictional force against the mover 18a1 when the mover 18a1 moves the needle unit 17, but is not limited to this. The cell puncture device 10 may not have the friction part 18e.

It is obvious to those skilled in the art that the present disclosure can be realized in other predetermined forms excluding the above-described embodiments, without departing from the spirit or the essential features thereof. Therefore, the foregoing descriptions are illustrative and not limited thereto. The scope of the disclosure is defined by the appended claims, not by the foregoing descriptions. Among any changes, some changes within the scope of equivalence thereof shall be included therein.

For example, the shapes, patterns, sizes, arrangements, orientations, types, numbers, and the like of the components described above are not limited to the contents of the descriptions and drawings above. The shapes, patterns, sizes, arrangements, orientations, types, numbers, and the like of the components may be configured arbitrarily as long as the functions thereof can be achieved. The illustrated components of the cell puncture device 10 and the microscope system 1 are conceptual in terms of functions, and the specific forms of the components are not limited to those illustrated.

The functions and the like included in each of the above-described components and the like can be rearranged so that there are no logical contradictions, and it is possible to combine multiple components or the like into one or split one component into multiple.

Examples of some embodiments of the present disclosure are described below. However, it should be noted that the embodiments of the present disclosure are not limited to these examples.
[Appendix 1] A cell puncture device comprising:
   a needle unit having a needle configured to puncture a cell, and a needle fixer configured to fix the needle;
   a needle support connected to the needle fixer, the needle support configured to arrange the needle unit at a distal end; and
   a first regulator arranged with respect to the needle support, the first regulator configured to locate the needle unit in a first position in which the needle punctures the cell,
   wherein the needle support includes a mover configured to move the needle unit between the first position and a second position to which the needle is evacuated.
[Appendix 2] The cell puncture device according to appendix 1, wherein
   the needle support has a support head configured to couple the mover and the needle fixer, and
   the first regulator is configured to locate the needle unit by contacting the support head.
[Appendix 3] The cell puncture device according to appendix 2, further comprising a base arranged opposite the support head with respect to the mover, the base being configured to move the first regulator between a third position for locating the needle unit in the first position and a fourth position different from the third position.
[Appendix 4] The cell puncture device according to appendix 3, further comprising a second regulator attached to the base, the second regulator being configured to regulate movement of the first regulator by the base.
[Appendix 5] The cell puncture device according to appendix 3 or 4, wherein the first regulator includes a protrusion that protrudes from a surface of the base toward the support head and that is adjacent to the mover.
[Appendix 6] The cell puncture device according to any one of appendices 3 to 5, wherein the first regulator is configured to detachably fix the support head by contacting the support head.
[Appendix 7] The cell puncture device according to appendix 6, wherein
   at least one of the first regulator or the support head has a magnet, and
   the first regulator is configured to detachably fix the support head by a magnetic force.
[Appendix 8] The cell puncture device according to appendix 6 or 7, wherein
   the mover is configured to rotatably move around a rotation axis, and
   the first regulator includes an opposite surface that is connected to a surface of the base and that intersects a rotation direction of the mover.
[Appendix 9] The cell puncture device according to appendix 8, wherein the first regulator has a positioning protrusion that protrudes so as to make point contact with the support head on the opposite surface facing the support head.
[Appendix 10] The cell puncture device according to appendix 1, wherein the first regulator is attached to the mover so as to penetrate the mover, and the first regulator is configured to locate the needle unit by moving together with the mover.
[Appendix 11] The cell puncture device according to appendix 10, further comprising a base having an engagement structure that is arranged opposite the needle unit with respect to the mover, and with which the first regulator engages in at least one of a fifth position for locating the needle unit in the first position or a sixth position different from the fifth position.
[Appendix 12] The cell puncture device according to appendix 11, wherein
   an end of the first regulator is hemispherical in shape, and
   the engagement structure includes an engagement groove whose cross-sectional shape tapers in a depth direction.
[Appendix 13] The cell puncture device according to appendix 11 or 12, wherein
   the base is configured to be rotatable so that the engagement structure rotatably moves, and
   the cell puncture device further comprises a second regulator that is arranged with respect to the base and that regulates rotation of the base.
[Appendix 14] The cell puncture device according to any one of appendices 1 to 13, further comprising a friction part configured to produce a frictional force against the mover when the mover moves the needle unit.
[Appendix 15] A microscope system comprising:
   the cell puncture device according to any one of appendices 1 to 14, and
   a microscope configured to image the cell that is to be punctured by the needle of the cell puncture device.

## Claims

1. A cell puncture device comprising:
a needle unit having a needle configured to puncture a cell, and a needle fixer configured to fix the needle;
a needle support connected to the needle fixer, the needle support configured to arrange the needle unit at a distal end; and
a first regulator arranged with respect to the needle support, the first regulator configured to locate the needle unit in a first position in which the needle punctures the cell,
wherein the needle support includes a mover configured to move the needle unit between the first position and a second position to which the needle is evacuated.

2. The cell puncture device according to claim 1, wherein
the needle support has a support head configured to couple the mover and the needle fixer, and
the first regulator is configured to locate the needle unit by contacting the support head.

3. The cell puncture device according to claim 2, further comprising a base arranged opposite the support head with respect to the mover, the base being configured to move the first regulator between a third position for locating the needle unit in the first position and a fourth position different from the third position.

4. The cell puncture device according to claim 3, further comprising a second regulator attached to the base, the second regulator being configured to regulate movement of the first regulator by the base.

5. The cell puncture device according to claim 3 or 4, wherein the first regulator includes a protrusion that protrudes from a surface of the base toward the support head and that is adjacent to the mover.

6. The cell puncture device according to claim 3 or 4, wherein the first regulator is configured to detachably fix the support head by contacting the support head.

7. The cell puncture device according to claim 6, wherein
at least one of the first regulator or the support head has a magnet, and
the first regulator is configured to detachably fix the support head by a magnetic force.

8. The cell puncture device according to claim 6, wherein
the mover is configured to rotatably move around a rotation axis, and
the first regulator includes an opposite surface that is connected to a surface of the base and that intersects a rotation direction of the mover.

9. The cell puncture device according to claim 8, wherein the first regulator has a positioning protrusion that protrudes so as to make point contact with the support head on the opposite surface facing the support head.

10. The cell puncture device according to claim 1, wherein the first regulator is attached to the mover so as to penetrate the mover, and the first regulator is configured to locate the needle unit by moving together with the mover.

11. The cell puncture device according to claim 10, further comprising a base having an engagement structure that is arranged opposite the needle unit with respect to the mover, and with which the first regulator engages in at least one of a fifth position for locating the needle unit in the first position or a sixth position different from the fifth position.

12. The cell puncture device according to claim 11, wherein
an end of the first regulator is hemispherical in shape, and
the engagement structure includes an engagement groove whose cross-sectional shape tapers in a depth direction.

13. The cell puncture device according to claim 11 or 12, wherein
the base is configured to be rotatable so that the engagement structure rotatably moves, and
the cell puncture device further comprises a second regulator that is arranged with respect to the base and that regulates rotation of the base.

14. The cell puncture device according to any one of claims 1 to 4 and 10 to 12, further comprising a friction part configured to produce a frictional force against the mover when the mover moves the needle unit.

15. A microscope system comprising:
the cell puncture device according to any one of claims 1 to 4 and 10 to 12, and
a microscope configured to image the cell that is to be punctured by the needle of the cell puncture device.
